(19) Europäisches Patentamt
European Patent Office
Office européen des brevets

(11) Publication number: 0 197 613
A2

(12) EUROPEAN PATENT APPLICATION

(21) Application number: 86200581.6

(51) Int. Cl.⁴: C08G 73/02 , B01J 27/18

(22) Date of filing: 04.04.86

(30) Priority: 04.04.85 US 720159

(43) Date of publication of application:
15.10.86 Bulletin 86/42

(84) Designated Contracting States:
BE DE FR GB IT NL SE

(71) Applicant: UNION CARBIDE CORPORATION
39 Old Ridgebury Road
Danbury Connecticut 06817(US)

(72) Inventor: Doumaux, Arthur Roy, Jr.
1401 Wilkie Drive
Charleston West Virginia 25314(US)
Inventor: Gibson, Charles Arnold
2910 Macon Street
So. Charleston West Virginia 25303(US)

(74) Representative: Smulders, Theodorus A.H.J. et al
Vereenigde Octrooibureaux Nieuwe Parklaan 107
NL-2587 BP 's-Gravenhage(NL)

(54) **Water addition to enhance phosphoric acid salt catalyst activity.**

(57) A method for enhancing the catalytic activity retention of a phosphorus acid salt catalyst, especially a metal acid phosphate during the production of polyalkylene polyamines from the reaction of a feed of (i) ammonia and/or an alkyleneamine compound having at least two amino groups, such as, ethylenediamine, and (ii) an alkanolamine having at least one amino group, such as, ethanolamine. Sufficient water is utilized in the feed to enhance the catalytic activity retention of the phosphorus acid salt catalyst. When a metal acid phosphate catalyst is used, the water keeps the metal acid phosphate in the acidic state and/or returns the metal acid phosphate to the acidic state. The reaction is conducted in the gas phase, in the presence of a catalytically effective amount of phosphorus acid salt catalyst and at a temperature sufficient to form the polyalkylene polyamines. Also, when the phosphorus acid salt catalyst has sustained a decrease in the activity during the production of polyalkylene polyamine, water can be added in sufficient amount to increase the activity of the catalyst.

EP 0 197 613 A2

# WATER ADDITION TO ENHANCE PHOSPHORIC ACID SALT CATALYST ACTIVITY

## BACKGROUND OF THE INVENTION

The invention relates to the preparation of polyalkylene polyamines.

U.S. Patent No. 4,463,193 discloses a process for preparing predominantly noncyclic polyalkylene polyamines. Ammonia or a primary or secondary amine is contacted with an alkanolamine compound having an amino group and a primary or secondary hydroxy group and an alkyleneamine compound having two amino groups in the presence of a catalytically effective amount of a Group IIIB metal acid phosphate. A temperature is used which is sufficient to effect a reaction among the ammonia or amine, the alkanolamine compound and the alkyleneamine compound under a pressure sufficient to maintain a substantial amount of the ammonia or amine in the reaction zone. U.S. Patent No. 4,463,193 does not have any teaching of the addition of water to the reaction zone.

U.S. Patent No. 4,044,053 discloses a process for preparing noncyclic polyalkylene polyamines which includes contacting an alkyleneamine compound having two primary amino groups with a diol having primary or secondary hydroxy groups of the general formula:

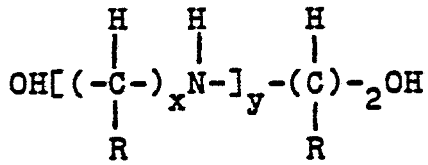

wherein R is a hydrogen or a lower alkyl; x is number from 2 to about 6; and y is a number from 0 to about 3 in the presence of a catalytically effective amount of a phosphorus-containing substance at a temperature of 250° to about 350°C. under a pressure sufficient to maintain the mixture essentially in liquid phase wherein the alkyleneamine compound is present in molar excess. The catalyst can be acidic metal phosphates, phosphoric acid compounds and their anhydrides, phosphorus acid compounds and their anhydrides, alkyl or aryl phosphate esters, alkyl or aryl phosphite esters, alkyl or aryl substituted phosphorus acids and phosphoric acids wherein said alkyl groups have from 1 to about 8 carbon atoms and said aryl groups have from 6 to about 20 carbon atoms, alkali metal monosalts of phosphoric acid and mixtures of the above. The polyalkylene polyamine compound is recovered from the resultant reaction mixture.

On the subject of water, U.S. Patent No. 4,044,053 states concerning its process: "It is not critical to control the amount of water of reaction present during the heating of reactants and catalyst, such as by removal thereof as it is formed. Usually, we prefer to retain the water in the reaction zone and remove it from the reaction mass during recovery of the predominantly non-cyclic polyalkylene polyamines." [Col. 6, lines 18 to 24].

The prior art section of U.S. Patent No. 4,044,053 states: "For example, U.S. Pat. No. 3,121,115 to Meuly teaches a process for aminoalkylating certain amines having a replaceable amino hydrogen, particularly aromatic primary and secondary amines, which includes heating the amine compound with an N-tertiary aminoalkanol at from 150° to 250°C. in liquid phase with continuous water removal in the presence of a phosphoric acid compound." [Col. 1, lines 55 to 63]

U.S. Patent No. 3,121,115 contains a disclosure for the production of aminoalkylated compounds containing tertiary amino groups. The disclosed process involves reacting alkylatable amines or phenols with N-tertiary aminoalkanols in the presence of a phosphoric acid catalyst. There is no disclosure of an alkylenediamine reactant, since the patent is limited to specifically describing monoamines or aromatic diamines; and there is also no disclosure of a difunctional alkylene glycol or alkanolamine reactant, since the patent is specifically limited to monofunctional, tertiary aminoalkanol reactants.

U.S. Patent No. 4,036,881 is very similar to U.S. Patent No. 4,044,053, except that U.S. Patent No. 4,036,881 uses (in place of the latter's diols) alkanolamine compounds having a primary amino group and a primary or secondary hydroxy group of the general formula:

$$H_2N-[(\underset{R}{\overset{H}{C}})_x\overset{H}{N}-]_y(-\underset{R}{\overset{H}{C}}-)_xOH$$

wherein R is a hydrogen or a lower alkyl radical; x is a number from 2 to about 6; and y is a number from 0 to 3. Similarly, U.S. Patent No. 4,036,881 discloses that it is not critical to control the amount of water of reaction present during the heating of reactants and catalyst, such as by removal thereof as the water is formed. Usually, such patent prefers to retain the water in the reaction zone and remove it from the reaction mass during recovery of the predominantly non-cyclic polyalkylene polyamines.

U.S. Patent No. 4,103,087 presents a process for reacting tertiary aminoalkanols with monofunctional secondary amines to produce a di-(N,N-disubstituted amino)alkane product. A heterogeneous alumina phosphate catalyst is used. U.S. Patent No. 4,103,087 states:

" It has been found advantageous in batch processes to maintain the water content of the reaction system at as low a level as is possible in order to enhance catalytic activity and simultaneously to shift the reaction equilibrium toward the desired product. Therefore, it is desirable to maintain the reaction zone at a pressure such that the water formed in the bimolecular condensation reaction will be removed from the reaction zone as a vapor." [Emphasis supplied] [Col. 6, lines 32 to 39]

European Patent Application No. 0069322 discloses the use of certain hydrogen phosphate and pyrophosphate catalysts and certain organic condensation reactions. In some of the disclosed catalytic reactions, presence of water in the reaction zone is disclosed; in some instances the water is added as a component of the reactor feed. On page 21 of such European Patent Application, diethyleneglycol was passed over a $SrHPO_4$ catalyst in the presence of water, the feed containing the diethyleneglycol in water. As further stated that $SrHPO_4$ is a highly selective catalyst. On page 22 concerning the same example, the European Patent Application states:

"The addition of water to the organic feeds may be desirable to prevent loss of catalyst activity as a result of dehydration of the $SrHPO_4$ to the pyrophosphate."

The above quotation appears to be in conflict with some of the primary teachings of the European Patent Application, namely page 4 thereof which states:

"The pyrophosphate form of the catalysts of the present invention are prepared by heating treating the metal monohydrogen or dihydrogen phosphate product at temperatures above about 300°C up to 750°C in the presence of a mixture of steam and air, preferably at least about 20% by volume of steam."

Page 4 specifically teaches that the pyrophosphate form of the catalyst can be prepared from the metal monohydrogen phosphate by treating them at a higher temperature in the presence of large amounts of steam and air. It is noted that apparently all of the hourly space velocity in the European Patent Application are liquid hourly space velocity which are measured as volumes of liquid per volume of catalyst.

Referring again to U.S. Patent No. 4,036,881, attention is drawn to Example II wherein an aqueous solution of ethylenediamine and a smaller amount of boron phosphate were charged to a reactor and placed under a nitrogen atmosphere. The reactor was run at 275° to 280°C. and at a pressure of 525 to 560 p.s.i.g. for two hours. The only polyamine present in the reactor mix was ethylenediamine. Example II specifically states: "*** indicating no reaction had occurred."

Metal acid phosphate catalysts tend to lose any meaningful activity after a relatively short time period of reaction. This means that the reactor must be shut down frequently to reactivate the catalyst. Also, the activity of metal acid phosphate catalysts drops rapidly during a run. The result is an economic loss due to the decreased conversion rate achieved in such reactors.

## BROAD DESCRIPTION OF THE INVENTION

The invention broadly involves enhancing or maintaining the activity of phosphorus acid salt catalysts used in the production of polyalkylene polyamines. More specifically, the invention provides means for prolonging the catalytic activity of a phosphorus acid salt catalyst during such gas phase reaction and also provides means for increasing the catalytic activity of a phosphorus acid salt catalyst which has partially lost activity during such reaction.

The invention involves a method for enhancing the catalytic activity retention of a phosphorus acid salt catalyst during the production of polyalkylene polyamines from the reaction of a feed of (i) of a reactive nitrogen-containing compound selected from the groups consisting of ammonia, a primary amine and a secondary amine, and/or an alkyleneamine compound having at least two amino groups and (ii) an alkanolamine having at least one amino group. The reaction is conducted in the gas phase, in the presence of a catalytically effective amount of a phosphorus acid salt catalyst and at a temperature sufficient to form the polyalkylene polyamines. Sufficient water is maintained in the feed to maintain the catalytic activity (enhance the catalytic activity retention) of the phosphorus acid salt catalyst during the course of the reaction. An important advantage of the invention is the fact that the increased catalyst life means that the reaction process can be run for much longer times without having to shut down the reactor to regenerate the catalyst. As is explained below, water can also be added during a reactor run to regenerate a catalyst that has reduced catalytic activity. Such procedure provides the advantage that the reaction process does not have to be stopped to regenerate a catalyst which has lost its activity during such continuous production run.

The reaction is thought to proceed, for example, by the reaction of ethylenediamine with monoethanolamine to form diethylenetriamine and a molar equivalent of water.

The catalyst used in the invention process can be any salt of a phosphoric acid that has amination catalytic action at high temperatures (as defined and specified herein). The water addition or inclusion in the reactor feed provides continuous hydrolysis of the phosphorus acid salt catalyst and prevents the formation of undesirable phosphoric amides and esters. Also, the hydrolysis prevents the phosphoric acid salts from being converted into pyrophosphates.

Enough water is added to the feed to provide the sufficient amount of water in the feed needed to maintain the catalytic activity. If, before the feed is fed into the reaction zone, the feed contains an amount of water which is insufficient, enough additional water can be added to the feed to provide the sufficient amount of water needed to maintain the catalytic activity.

Advantageously, the water, ammonia, alkylenemine compound and alkanolamine compound can be mixed and fed into the reaction zone. Also, when the ammonia, alkyleneamine compound and alkanolamine compound are separately fed into the reaction zone, advantageously enough water is added to the ammonia or the alkyleneamine compound or the alkanolamine compound to provide the sufficient amount of water in the feed needed to maintain the catalytic activity.

Preferably the amount of water used in the feed is 1 to 50 weight percent, based on the total weight of the alkyleneamine compound and the alkanolamine compound, and most preferably the amount of water used in the feed is 10 to 25 weight percent, based on the total weight of the alkyleneamine compound and the alkanolamine compound. When an alkyleneamine is not in the feed, then such water weight percentages are based only on the alkanolamine. The invention process provides excellent results when the alkyleneamine compound is ethylenediamine and the alkanolamine compound is ethanolamine.

A particularly advantageous embodiment of the invention process is where water, which has been separated from the reaction mixture resulting from the reaction, is recycled and added to the feed to provide the sufficient amount of water in the feed needed to maintain the catalytic activity. Preferably the separated water is in the form of an azeotropic mixture of (a) water and (b) the alkyleneamine compound.

Preferably the phosphorus acid·salt catalyst is a metal acid phosphate catalyst. In such instance, sufficient water is maintained in the feed to keep the metal acid phosphate in the acid state. One group of preferred catalysts are the Group IIIB metal phosphates, Group IIIB metal monohydrogen phosphates and Group IIIB metal dihydrogen phosphates supported on an inert carrier. Somewhat high reaction temperatures are used with the Group IIIB metal acid phosphate catalysts. The catalyst can be impregnated on a support material which is alumina, silica, silica-alumina, diatomaceous earth or silica-titania.

The invention process is preferably conducted so that the reaction occurs in the vapor phase or supercritical phase. For purposes herein, the term gas phase encompasses both the vapor phase and supercritical phase. The reaction is conducted at a pressure of about 50 to 4,000 p.s.i.g. The reaction is best conducted at a temperature high enough to keep the reactants above their dew points. This may also mean at a pressure (first defined by temperature) which expresses the first quadrant using the critical point as the origin. The pressure in the reaction zone is preferably between 100 and 2,000 p.s.i.g. A nonaqueous, gaseous diluent can also be fed into the reaction zone. Examples of suitable diluents are methane, hydrogen, nitrogen or argon. The liquid hourly space velocity (pounds of feed per pound of catalyst per hour) of the

reactants is between about 0.1 and about 100 per hour and preferably between 1 and 25 $hr^{-1}$. Preferably the reaction is conducted at a temperature between 220° and 350°C. and most preferably at 260° to 300°. Also preferably the reaction is conducted as a continuous reaction.

The invention also involves a method for increasing the catalytic activity of a phosphorus acid salt catalyst during the production of polyalkylene polyamines from the reaction of a feed of (i) of a reactive nitrogen-containing compound selected from the groups consisting of ammonia, a primary amine and a secondary amine, and/or an alkyleneamine compound having at least two amino groups and (ii) an alkanolamine having at least one amino group. The reaction is conducted in the presence of a catalytically effective amount of a phosphorus acid salt catalyst and at a temperature sufficient to form the polyalkylene polyamines. This embodiment of the invention involves the situation where the catalytic activity of phosphorus acid salt catalyst has decreased during the course of the reaction. Sufficient water is then included in the feed to increase or enhance the catalytic activity of the phosphorus acid salt catalyst at that point in the reaction. Preferably enough water is included in the feed to substantially restore the catalytic activity of the catalyst to the level it had at the start of the reaction. Also it is preferable to thereafter maintain sufficient water in the feed to maintain the catalytic activity of the phosphorus acid salt catalyst during the remainder of the reaction.

## DETAILED DESCRIPTION OF THE INVENTION

As used herein, all parts, percentages, ratios and proportions are on a weight basis and all temperatures are in degrees Centigrade, unless otherwise stated or otherwise obvious herefrom. Also as used herein, the term "ethanolamine" specifically means monoethanolamine unless otherwise indicated or implied herein. U.S. Sieve Series mesh sizes are used herein.

The alkyleneamine having at least two amino groups is preferably an unbranched alkylene moiety, such as, ethylenediamine, and preferably has primary amino groups. The alkanolamine preferably has a primary or secondary hydroxy moiety and preferably a primary amino group(s). Preferably, the alkanolamine has an unbranched alkylene moiety.

The alkanolamine compounds used in the invention process include those represented by the formula:

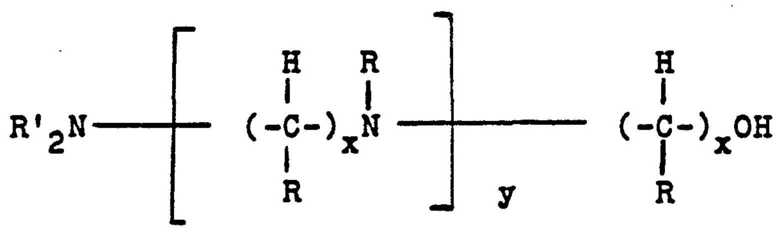

wherein R is hydrogen or a lower alkyl ($C_1$ to $C_4$) radical, R' is hydrogen or an alkyl ($C_1$ to $C_{25}$) radical, x is a number from 2 to 6, and y is a number from 0 to 3. Examples of suitable alkyl radicals are the lower ($C_1$ to $C_4$) alkyls, such as, methyl, ethyl and butyl, and higher alkyls, such as, octyl, decyl and octadecyl. Methyl is the preferred lower alkyl radical. However, it is preferred that R and R' both are hydrogen; thus the alkanolamine would contain a primary amino group. Examples of useful alkanolamine compounds are the ethanolamines, isomeric propanolamines, N-(2-aminoethyl) ethanolamine, N-methylethanolamine, N,N-dimethylethanolamine, N,N,N'-trimethylaminoethylethanolamine and the like. Recycle of at least a portion of the unreacted alkanolamine recovered from the product stream to the reactor allows control of the molecular weight distribution and range of the produced polyalkylene polyamines.

The alkyleneamine reactants used in the invention process are represented by the formula:

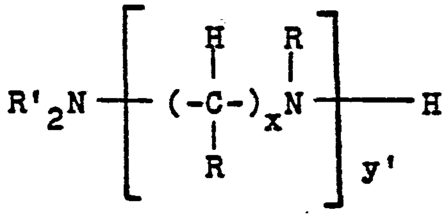

wherein R is hydrogen or a lower alkyl ($C_1$ to $C_4$) radical, R' is hydrogen or an alkyl ($C_1$ to $C_{25}$) radical, x' is a number from 2 to 6, and y' is a number from 1 to 4. Examples of suitable alkyl radicals are the lower ($C_1$ to $C_4$) alkyls, such as, methyl, ethyl and butyl, and higher alkyls, such as, octyl, decyl and octadecyl. It is preferred that R and R' are both hydrogen. The preferred lower alkyl radical is methyl. Examples of useful alkyleneamine compounds are 1,3-propylenediamine, N-methylpropylenediamine, 1,2-propylenediamine, diethylenetriamine, tributylenetetraamine, triethylenetetraamine, N,N,N'-trimethyldiethylenetriamine, noncyclic isomers of triethylenetetraamine, noncyclic isomers of tetraethylenepentamine, N-methylethylenediamine, N,N-dimethylethylenediamine and ethylenediamine, which is the preferred alkyleneamine compound. Recycle of at least a portion of the alkylenediamine separated from the product stream to the reactor allows control of the molecular weight distribution and range of the produced polyalkylene polyamines.

Generally, the mole ratio of alkyleneamine compound to alkanolamine compound can range from about 0.05:1 to 12:1, and preferably is about 1:4 to 4:1.

The invention process predominantly produces noncyclic polyalkylene polyamines. Noncyclic polyalkylene polyamines that are generally produced by the reaction of ammonia and alkanolamine (plus an alkylenediamine) are represented by the formula:

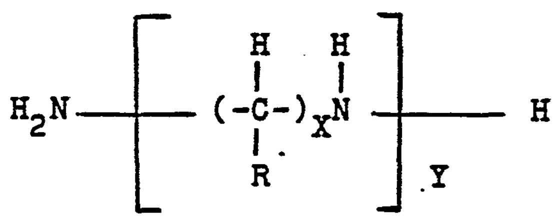

wherein R is hydrogen or a lower alkyl ($C_1$ to $C_4$) radical, preferably a methyl radical, X is a number from 2 to 6, Y is a number from 2 to 7, and X can vary for a given value of Y. Examples of such noncyclic polyalkylene polyamines that are produced are dipropylenetriamine, tributylenetetramine, di(2-methylethylene)triamine, tri(2-methylethylene)tetramine, N-(2-aminoethyl)-1,3-propylenediamine, diethylenetriamine, the noncyclic isomers of triethylenetetramine and the noncyclic isomers of tetraethylenepentamine. It is desirable not to coproduce significant amounts of byproducts, particularly cyclic amines, especially piperazine and aminoethylpiperazine, that are of less commercial value than diethylenetriamine.

Noncyclic polyalkylene polyamines include polyalkylene polyamines having straight-chained and/or branched alkylene groups.

Cyclic polyalkylane polyamines that are produced by the reaction of (a) an alkyleneamine and an alkanolamine or (b) ammonia, an alkyleneamine and an alkanolamine or (c) an alkanolamine and ammonia are represented, for example by the following formula:

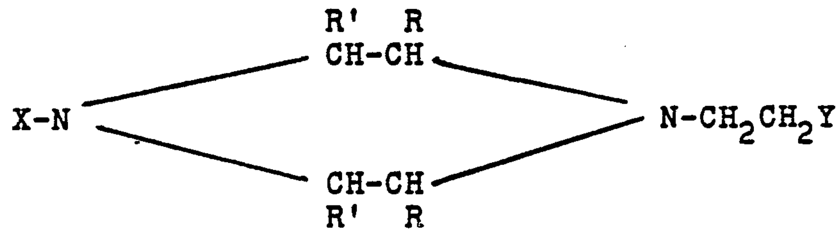

wherein R is hydrogen, an alkyl group containing 1 to 12 carbon atoms or a cycloalkyl group containing 6 to 12 carbon atoms, R' is hydrogen or an alkyl group containing 1 to 4 carbon atoms, X is hydrogen or $-CH_2CH_2Y$ and Y -OH or $-NH_2$.

Examples of cyclic polyalkylene polyamines are pierazine, N-(2-hydroxyethyl)piperazine, N,N-di-(hydroxyethyl)piperazine, N-(hydroxyethyl)-2-methylpiperazine, N,N'-di(hydroxyethyl-2-methylpiperazine, N-(hydroxyethyl)-2-ethylpiperazine, N,N'-di(hydroxyethyl)-2-ethylpiperazine, N-(hydroxyethyl)-2-butylpiperazine, N,N-di-(hydroxyethyl)-2-butylpiperazine, N-(hydroxyethyl)-2-dodecylpiperazine, N-(hydroxyethyl)-2-cyclohexylpiperazine, N-(hydroxy ethyl)-2-hexylcyclohexylpiperazine, N,N'-(dihydroxyethyl)-2,5-dimethylpiperazine, N-(hydroxyethyl)-2,3,5,6-tetramethylpiperazine, N,N'-di(hydroxyethyl)-2,5-dimethylpiperazine, N-(hydroxyethyl)-2,5-diethylpiperazine, N-(hydroxyethyl)diethylenetriamine, N-(hydroxypropyl)diethylenetriamine, N-(2-hydroxypropyl)diethylenetriamine, N-(2-hydroxybutyl)-diethylenetriamine, N-(hydroxyethyl)-diproprylenetriamine, N-(hydroxypropyl)-dipropylenetriamine, N-(2-hydroxybutyl)-dipropylenetriamine and morpholine.

The phrase "predominantly noncyclic polyalkylene polyamines" is meant to mean that such polyalkylene polyamines are mostly of the noncyclic species.

In the reactor, the temperature for the reaction depends upon the particular starting material, ratios of reactants, and most importantly, the activity of the catalyst used. Generally, in processes of the invention, temperatures within the range of 125° to 425°C. are suitable while a preferred range is 220° to 350°C. and the most preferred range is 260° to 300°C. Diluent gas can be utilized to help in the control of the reaction temperature and assist in maintaining the desired pressure.

A relatively high pressure of the reaction is also preferred. Normally, a nonaqueous diluent, such as, hydrogen, helium, nitrogen, methane or argon, can be added to increase the pressure in the reactor. The pressure at the time of reaction should normally be within the range from about 50 to about 4,000 p.s.i.g., preferably greater than 100 p.s.i.g., and most preferably from 200 to about 2,000 p.s.i.g. The reaction is best conducted at a temperature high enough to keep the reactants above their dew point. This may also mean at a pressure (first defined by temperature) which expresses the first quadrant using the critical point as the origin.

The reaction is preferably conducted in the vapor phase, but can be conducted in the supercritical phase. The liquid hourly space velocity of the reactants (EDA and MEA or EDA, MEA and $NH_3$) is between about 0.1 and about 100 per hour and preferably between about 1 and about 25 per hour.

By reaction zone is meant that vessel, e.g., autoclave, continuous stirred tank reactor or packed bed reactor, in which the catalyst is located and production of polyalkylene polyamines is effected.

Although the reactions can be carried out in the batch mode, they are preferably conducted as continuous processes, for example, operation of a continuously stirred tank reactor or a packed (fixed) bed reactor. The continuous process is carried out by employing conventional process techniques and apparatus well known to those skilled in the art. In the continuous reaction processes, the catalyst can be added alone or in combination with the reactants, or, as stated above, the catalyst can be provided as a fixed bed on conventional support materials well known to those skilled in the art. The reaction is allowed to proceed until a desired conversion is obtained or the reaction is complete. Normally the reaction is carried out within about 0.5 to 5 hours in the batch mode or residence times - (based on the alkanolamine and alkyleneamine components) of 0.1 to 4.0 hours in a continuous mode for practical levels of polyalkylene polyamine production.

The reactor can be an up-flow or down-flow reactor and can have a fluidized bed or, most commonly, a fixed bed. The catalyst bed can contain inert particles which can be interspersed throughout the bed and/or form discrete layers, e.g., at an end or intermediary to the bed. The volume of the reaction zone containing such inert

particles is the reaction zone volume for purposes of determining the feed rate. Preferably, the space velocity should not be so high that for the reactor geometry, a significant amount of backmixing occurs. Advantageously, the flow through the catalyst bed is substantially plug-type flow.

Ammonia or a primary amine or secondary amine can also be used in the feed to the reactor. The ammonia is both a reactant and a diluent - (when excess of it is present) in the invention process. When ammonia is used, the mole ratio of ammonia (or the primary amine or the secondary amine) to the combination of ethylenediamine and monoethanolamine is usually in the range of 20:1 to 0.6:20 and preferably in the range of 12:1.25 to 1:1. The ratios apply also to where the feed only contains ammonia (or amine) and monoethenolamine. Ammonia and the preferred primary and secondary amines which are used in the invention process are represented by the formula:

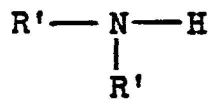

wherein R' is hydrogen or an alkyl ($C_1$ to $C_{25}$) radical, preferably a lower alkyl ($C_1$ to $C_4$) radical, such as methyl or ethyl. Stoichiometrically, one molecular unit of ammonia or amine (primary or secondary) is required per molecular unit of hydroxyl group. However, the formation of diethylenetriamine is favored by the presence of excesses of ammonia, but a practical limit on the amount of ammonia employed exists due to energy consumption. Useful amine feedstocks include monomethylamine, dimethylamine, monoethylamine, diethylamine, octylamine and octadecylamine.

Use of secondary amines instead of ammonia leads to polyalkylene polyamines containing terminal dialkylamino groups. Alternatively, use of primary amines instead of ammonia leads to polyamines which contain randomly distributed monoalkylamino groups.

The catalysts used in the invention are heterogeneous catalysts. The catalysts are used in an amount of 0.1 to 12 weight percent, preferably 0.5 to 10 weight percent, and most preferably 2 to 7 weight percent, based on the total weight of the reactants.

The catalyst used in the invention process can be any salt of a phosphorus acid that has amination catalytic action at high temperatures (as defined and specified herein). The water addition or inclusion in the reactor feed provides continuous hydrolysis of the phosphorus acid salt catalyst and prevents the formation of undesirable phosphorus amides and esters. Also, the hydrolysis prevents the phosphorus acid salts from being converted into pyrophosphates. Within such parameters, generally any phosphorus acid salt catalyst can be used. One group of preferred phosphorus acid salt catalyst are the metal phosphate catalysts, which can be metal phosphates, metal monohydrogen phosphates and metal dihydrogen phosphates.

The metal phosphate catalysts include boron phosphate, aluminum phosphate, ferric phosphate, zinc phosphate, ferrous phosphate, nickel phosphate, chromium phosphate, copper phosphate and cobalt phosphate. Other metal phosphate catalysts which can be used are the phosphates of lithium, sodium, potassium, other metals of Group IA of the periodic tables, beryllium, magnesium, calcium, other metals of Group IIA of the periodic tables, titanium, zirconium, or other metals of Group IVB of the periodic table, antimony and tin (valence states II and IV). Further useful catalysts are those phosphoric acid salts which comprise a phosphorus bonded to a Group IVB transition metal oxide support, such as is disclosed in published European Patent Application 0115138 (the pertinent parts of which are incorporated herein by reference). Mixtures of two or more of the metal phosphate catalysts can be used.

The metal phosphate catalysts also include the pyrophosphates, monohydrogen phosphates and dihydrogen phosphates of strontium, copper, magnesium, calcium, barium, zinc, aluminum, cobalt, nickel cerium, neodymium, and mixtures thereof. Specific examples of such catalysts are $SrHPO_4$, $Sr/BaHPO_4$, $Sr(H_2PO_4)_2$, $Ca(H_2PO_4)_2$, $Nd_2(HPO_4)_3$, $Ce_2(HPO_4)_3$, $CoHPO_4$, $NiHPO_4$, $Al_2(HPO_4)_3$, $MgHPO_4$, $BaHPO_4$, $CuHPO_4$ and $ZnHPO_4$.

The metal phosphate catalysts include crystalline zirconium phosphate (U.S. Patent No. 3,416,884) and granular zirconium phosphate (U.S. Patent No. 4,025,608) -the pertinent parts of such patents are incorporated herein by reference.

The metal phosphate catalysts which are most preferred for practicing the process of the invention are Group IIIB metal acid phosphates including Group IIIB metal phosphates, monohydrogen phosphates, dihydrogen phosphates and mixtures thereof. U.S. Patent No. 4,463,193 (the pertinent parts of such patent are incorporated herein by reference)

discloses processes for preparing the Group IIIB metal acid phosphates. While the intent of the catalyst preparation is to specifically provide a particular Group IIIB monohydrogen phosphate or dihydrogen phosphate, mixtures of the Group IIIB metal phosphates of the above-mentioned types may be obtained owing to complicated dependence of the catalyst composition on preparation conditions. Nevertheless, although the Group IIIB metal acid phosphate catalyst of the invention comprises the metal phosphate, monohydrogen phosphate, dihydrogen phosphate or mixtures thereof, the monohydrogen and dihydrogen phosphates of the Group IIIB metals are the preferred catalysts when in relatively pure form individually or in combination.

A Group IIIB metal is meant to include scandium, yttrium, lanthanum and the rare earth lanthanide metals having atomic numbers 58 to 71, and the rare earth actinides having atomic numbers 89 to 92.

The most preferred metal phosphate catalysts for the production of noncyclic polyalkylene polyamines are the acid phosphates, preferably the monohydrogen phosphates and dihydrogen phosphates, of scandium, lanthanum, cerium, samarium, europium, thulium, erbium, ytterbium, yttrium, lutetium, thorium, neodymium, praseodymium, dysprosium and gadolinium.

The acid phosphate catalysts can be used for the production of polyalkylene polyamines either singly or in combination.

It is preferred to use those which are more catalytically active and provide for substantial conversion to the noncyclic polyalkylene polyamine products. Examples of the most preferred catalyst compounds include lanthanum monohydrogen phosphate, lanthanum dihydrogen phosphate, lanthanum phosphate, praseodymium monohydrogen phosphate, praseodymium dihydrogen phosphate, praseodymium phosphates, neodymium monohydrogen phosphate, neodymium dihydrogen phosphate, neodymium phosphate and mixtures thereof.

The quantity of the acid phosphate salts of the Group IIIB metals used in the reaction can vary widely depending upon the reactivity of the catalysts and the reactivity of the reactants present. A catalytically effective amount of material is used; in other words, an amount which causes a reaction, the alkyleneamine compound and the alkanolamine compound to yield polyalkylene polyamine products at the temperature and pressure used. Usually though, the amount used to provide a catalytic effect ranges from about 0.1 to 25 mole percent based upon the total amount of alkyleneamine compound and alkanolamine compound feed present in the reaction mixture, and preferably is an amount of about 0.1 to 10 mole percent. Within these ranges though, the level of catalyst is empirical and is adjusted depending on the product slate desired.

The Group IIIB metal phosphate catalysts used in the process of the invention can be prepared by the precipitation of the desired metal acid phosphate salt, washing the salt to remove inorganic coproducts and drying the salt. Optionally, dried catalysts can be further processed prior to use for polyalkylene polyamine manufacture.

Such process is well known to those skilled in the art and includes extrusion or pelletizing or compounding with an inert support such as alpha-alumina.

Methods of preparing Group IIIB metal monohydrogen phosphate or dihydrogen phosphate are disclosed in U.S. Patent No. 4,324,917 (the pertinent parts of the patent are incorporated herein by reference). Phosphoric acid salt materials can be obtained which consist predominantly of the Group IIIB metal phosphate, the Group IIIB metal monohydrogen phosphate, the Group IIIB metal dihydrogen phosphate, or mixtures in varying proportions of the Group IIIB metal monohydrogen and dihydrogen phosphate, and/or mixtures in varying proportions of any of the above Group IIIB metal acid phosphates with the Group IIIB metal phosphate. Such variations in catalyst composition can result from dependence of the catalyst composition on preparation conditions, such as temperature, concentration of reagents, stoichiometry of reagents, rate and order of reagent additions, pH of preparation, duration of preparation, volume and pH of water wash, duration of catalyst washing, and duration and temperature of catalyst drying. In any event, the Group IIIB metal acid phosphates obtained according to the general preparations referred to above are catalytically active for the production of polyalkylene polyamines.

Published European Patent Application 0115138 (the pertinent parts of which are incorporated herein by reference) discloses method for preparing phosphoric acid salt catalysts comprising a phosphorus bonded to a Group IVB metal oxide support. Any appropriate liquid or liquefiable phosphorus compound can be used as a source of the phosphorus. Useful phosphorus compounds include phosphoric acid, phosphorus acid, polyphosphoric acid, alkyl phosphates and alkyl phosphites such as trimethyl phosphate, triethyl phosphate, trimethyl phosphite, triethyl phosphite, etc. may be utilized. Also, a diaminohydrogen phosphate such as diammonium hydrogen phos-

phate, $(NH_4)_2HPO_4$, dimethyldiamino hydrogen phosphate, diethylamino hydrogen phosphate, etc. may be used. The catalyst compositions are prepared by depositing a phosphorus compound on a support comprising an oxide of a group IVb transition metal oxide. The group IVb transition metal oxides include the oxides of titanium, zirconium, hafnium and thorium. Pellets of the group IVb metal oxide may be prepared by extrusion or by compaction in conventional pelleting apparatus using a pelleting aid such as graphite. The phosphorus compound can be deposited on a powdered IVb metal oxide followed by pelleting and calcination. Preferably the catalyst composition is prepared by impregnating a preformed pellet. A suitable procedure to be used is to heat a liquid containing the liquid or liquefiable phosphorus compound at a temperature of about 100° to about 150° and to then add pellets in an amount about equal to the volume of the heated liquid. This treatment should be continued from about 0.5 to about 5 hours. At the end of that time, the resulting mixture of pellets and liquid is cooled, decanted to remove excess liquid followed by washing with an amount of water adequate to substantially completely remove unadsorbed liquid. Temperatures above 150°C can be used, if desired, but there is no particular advantage in doing so. It will be understood that the phosphorous that is present on a thus-treated pellet is not present as elemental phosphorous, but rather as phosphorous that is chemically bound, probably as an oxide, to the group IVb metal oxide support. However, the exact nature of the bonding is not completely understood.

European Patent Application 0115138 discloses amount of phosphourous that is bonded or otherwise adheres to the support is a function of heating and other conditions used in the treating step and is also a function of the chemical identity of the phosphorous compound that is used as a source of phosphorus. Under the treating conditions exemplified above, at least about 2.5 weight percent of phosphorus is caused to bond or otherwise permanently adhere to the pellets. There is an upper limit to the amount of phosphorus that bonds or otherwise permanently adheres to the support. This upper limit is, as indicated, a function of both the treating conditions and the chemical used as a source of the phosphorus. Normally, the maximum amount of phosphorus that can be caused to bond or otherwise permanently adhere to the pellets is within the range of about 5 to 10 weight percent. As a matter of convenience, the normal practice is to use only one chemical as a phosphorus source - (e.g., phosphoric acid). However, mixtures of two or more such reagents may be used, if desired. Calcining is not mandatory if the pellets are impregnated at least about 100°C, but the pellets can be calcined, if desired. Calcining is conducted for 2 to 24 hours at a temperature of 100°C or more but below the temperature at which thermal destruction of the phosphorus bonding occurs. This can be determined experimentally for a particular catalyst. Temperatures above 900°C, should be avoided. A suitable calcining temperature range is normally 200° to 800°C and, more preferably 500° to 700°C. Other procedures can be used in adding phosphorous to the group IVb metal oxide.

The pertinent parts of copending, commonly-assigned U.S. Application Serial No. 576,807, filed on February 7, 1984, are incorporated herein by reference. U.S. Serial No. 576,807 discloses certain phosphorus acid salt catalysts within the scope of the invention herein, provided the ester or amide derivatives are not used. The term phosphorus acid or acid derivative defines compounds having a P-X bond wherein P is a phosphorus atom bonded to a halogen, oxygen, sulfur or nitrogen atom is X which is a radical capable of hydrolyzing to produce the corresponding phosphorus acid structure.

Phosphorus-containing compounds such as trialkyl and triaryl phosphines and phosphine oxides, which contain no such exchangeable substructure, do not function as catalysts as defined in U.S. Serial No. 576,807. Very sterically hindered phosphorus compounds such as hexaethyl phosphoric triamide, while containing the requisite exchangeable substructure and functioning to some extent, are less preferred catalysts because they undergo the exchange process with the alkanolamine or alkylene glycol hydroxyl moieties only slowly. Phosphorus acids are defined by those structures wherein X in the P-X radical is a hydroxyl radical. Acid derivatives are defined by structures wherein X is a substitute functional group. Various suitable acid derivatives are salts when -X is $-O^-M^+$ is a mono or polyvalent cation. The precise phosphorus acid salt derivative structure is not critical so long as it fulfills the following two functional requirements: (1) that it provides for the relatively selective production of predominantly linearly extended polyalkylene polyamines and (2) that it enables increased conversion rates for polyalkylene polyamine production when water is removed during the reaction, possibly due to the water-inhibited formation of a phosphorus intermediate compound during the reaction.

The phosphorus acid salt derivative catalysts of U.S. Ser. No. 576,807 include those having the structure:

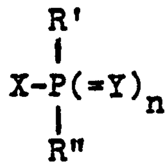

wherein Y is an oxygen or sulfur atom; n is 0 or 1; X is hydroxy, alkoxy, aryloxy, or the thioanalogs of the foregoing, alkyl or aryl substituted amino, halo, or the salts of the foregoing when X is hydroxy or mercapto; R' and R" are hydrogen, alkyl, aryl or one of the groups previously defined by X.

Suitable phosphorus acid salt derivatives of U.S. Ser. No. 576,807 which can be employed include, for example, acidic metal or semi-metal phosphates, phosphoric acid compounds, alkyl or aryl phosphates, alkyl or aryl phosphites, alkali metal monosalts of phosphoric acid, and mixtures of any of the above. Suitable acidic metal or semi-metal phosphates include boron phosphate, ferric phosphate, aluminium phosphate and the like. Any commercially available mono-, di-, or trialky or aryl phosphate or phosphate ester can be employed. In addition, bis-(phosphates), such as those disclosed in U.S. Patent No. 3,869,526, can be utilized. Preferred phosphorus catalyst include boron phosphate, ferric phosphate, aluminium phosphate, phosphoric acid and phosphorous acid.

The amount of phosphorus acid salt acid derivative catalyst of U.S. Ser. No. 576,807 utilized is a catalytically effective amount to cause condensation of the reactants to produce predominantly diethylenetriamine. This quantity will vary depending upon the reaction conditions and catalyst utilized. Usually a catalytically effective amount will be from about 0.01 to about 10 mole percent and preferably from about 1 to about 3 mole percent, based on the moles of hydroxy alkylene compound used.

Any of the phosphorus acid salt catalysts useful in the invention can be supported on carriers, such as, silica, silica-alumina, silica-titania, alumina, diatomaceous earth (kieselguhr) and any other conventionally employed inert reactor packing material. Generally, the catalysts are supported. The active catalyst species are provided on the surface of the support through, for example, coating or impregnation. The catalyst (say metal) components on the support often comprise about 1 to 50, say, about 3 to 30, weight percent of the catalyst. Useful supports can be porous and have surface areas of from about 0.1 to 500, say, about 0.3 to 100, square meters per gram.

The catalyst can be of any convenient size or shape. Catalysts can be made in the form of powders, spherical or conical pellets, extruded strips and the like. Impreganted spherical pellets ranging in diameter from 1/8 inch to 3/16 inch and extruded strips of a cylindrical-type shape ranging from 1/32 inch to 1/2 inch in length are typical of those which can be used as supports. Often, for commercial-scale operations, the pellets range in diameter from about 0.1 to 1 centi meter.

As the reaction process progresses, the phosphorus acid salt catalyst is normally deactivated and after a period of time it needs to be regenerated. The use of the invention, namely, the addition of water to the feed going into the reaction zone, can greatly prolong the life of the catalyst so that a high level of catalytical activity is achieved over a greatly extended period of time. The invention is further of use in adding water to a feed after a catalyst has lost some of its activity during a reaction run. In such instance, the catalyst can be brought back up to substantially its initial level of activity. By adding water thereafter on a continuous basis to the feed the regenerated catalyst activity level can be maintained for a very long time (much longer than the normal catalyst life when the addition of water in made).

The level of water that is added in the feed is preferably at a level of 1 to 50 weight percent, most preferably about 10 to 25 weight percent, based upon the total weight of the alkyleneamine and the alkanolamine. The water can be added into the feed and the components can be admixed, or the water can be admixed with one of the reactants and fed into the reaction zone, or the reactants and water can all be separatly added to the reaction zone. It is possible to admix the water intermittently with the alkyleneamine and/or the alkanolamine and feeding such mixture(s) into the reaction. It is by far more advantageous to add sufficient water on a continuous basis to the feed so as to maintain the catalyst at its highest level of activity during the entire length of the reaction run.

Recovery of the polyalkylene polyamines, alkanolamine and alkylenediamine from the reaction mixture from the reactor can be accomplished by conventional techniques.

Preferably the separation step is conducted using distillation. Most preferably the separation is conducted using a fractional distillation column with the polyethylene polyamines coming off of the bottom of the column and with the unreacted alkanolamine and alkylenediamine coming off of the top portion of the column. With a fractional distillation column, for example, having 10 trays, a pressure of 1000 p.s.i.g. and a base temperature of 220°C., 90 mole percent of the unreacted ethanolamine and ethylenediamine come off of the top of the column with the unreacted $NH_3$ and $H_2$, and 90 mole percent of the diethylenetriamine comes off of the bottom of the column.

The separation can be conducted in adsorbers, with the adsorbing liquids removing the polyalkylene polyamines from unreacted alkanolamine and alkylenediamine. Preferably the adsorbing liquid for the polyalkylene polyamines is triethylenetetraamine or a higher boiling adsorbent.

The separation can also be conducted using a series of partial condensers. The alkanolamine and alkylenediamine condense out in the last of the partial condensers, and the polyalkylene polyamines condense out in the first of the partial condensers. Preferably 3 to 5 partial condensers are used.

During separation of the various components in the product feed coming out of the reaction zone, water can also be separated out and recycled. Most preferably the recycled water is in the form of an azeotrope with the unreacted alkyleneamine. By recycling the water in the form of an azeotrope with one or both of the unreacted components, considerable energy is saved in the separation step.

Polyethylene polyamines are useful as corrosion inhibitors, fabric softeners, lubricating oil additives, co-monomers for polyamide resins, fungicides, surfactants, curing agents for epoxy resins and chelating agents.

Copending, commonly-asigned patent application entitled "Interreactor Separator", (D-14871), concurrently filed with this application, is incorporated herein by reference. Such application discloses the use of a reductive amination reaction zone from which its effluent is separated into a, preferably, gaseous, MEA-and EDA-containing phase and a liquid, DETA-rich phase. The gas phase can be used for recycle (advantageously), it is at high pressure and suitable for recycle without undue energy penalties) or as a feed to another reactor which can use a reductive amination or other type (e.g., phosphorus-based catalyst) of catalyst.

Copending, commonly-assigned patent application entitled, "Improving The Quality Of Catalytically Prepared Polyalkylene Polyamines", (D-14872), concurrently filed with this application, is incorporated herein by reference. Such application discloses processes for making polyalkylene polyamines from ethanolamines and a nitrogen compound (e.g., ammonia or alkyleneamine) using a phosphorus-based catalyst in the presence of sufficient hydrogen to enhance color and reduce odor.

Copending, commonly-assigned patent application entitled "Conversion Of Oxygen-Containing Polyamines", (D-14873), concurrently filed with this application, is incorporated herein by reference. Such application discloses, in its broadest aspect, passing an oxygenated, polyethylenepolyamine feed in the presence of a nitrogen compound to a reaction zone containing a phosphorus-based catalyst. Advantageously, this feed is obtained from a polyethylenepolyamine reactor having a reductive amination catalyst. The feed typically contains AEEA.

Copending, commonly-assigned patent application entitled "Two Reactor Scheme Using Only Phosphorus-Containing Catalysts", (D-14874), concurrently filed with this application. Such application discloses passing an oxygenated, polyethylenepolyamine feed in the presence of a nitrogen compound to a reaction zone containing a phosphorus-based catalyst. This feed is obtained from a polyethylenepolyamine reactor having a phosphorus-based catalyst. The feed typically contains AEEA.

Copending, commonly-assigned patent application entitled "Interchangeable Process Scheme", - (D-14875), concurrently filed with this application, is incorporated herein by reference. Such application discloses processes for making and separating ethyleneamines, alkylamines, morpholine, etc., in the same proces equipment. The separation systems required for these processes are very similar. Hence, block operation in the same reactor and separation equipment is achieved.

Copending, commonly-assigned patent application entitled "Preparation of Diethylenetriamine With Azeotrope Recycle", (D-14876), concurrently filed with this application, is incorporated herein by reference. Such application discloses processes for making ethyleneamines using a reductive amination or phosphorus-based catalyst in which a gaseous EDA/water phase is separated from the reactor effluent and at least a portion of the separated

phase is returned to the reactor. In a preferred embodiment using a reductive amination catalyst, the recycle stream is admixed with MEA to break the azeotrope with water condensing out.

Copending, commonly-assigned patent application entitled "Use of Pressure To Control the Noncyclics/-Cyclics Ratio of Polyalkylene Polyamines", (D-14878), concurrently filed with this application, is incorporated herein by reference. Such application discloses processes for producing polyalkylenepolyamines over phosphorus-based catalysts in which control of the ratio of noncyclic to cyclic products is achieved by controlling the level of the reaction pressure.

The following compound abbreviations apply herein:

EDA -ethylenediamine

MEA - monoethanolamine

PIP -piperazine

AEP -aminoethylpiperazine

DETA -diethylenetriamine

TETA(NC) -treithylenetetramine (noncyclic isomers)

TETA (C) -triethylenetetramine (cyclic isomers)

TEPA(NC) -tetraethylenepentamine (noncyclic isomers)

TEPA(C) -tetraethylenepentamine (cyclic isomers)

HVY (NC) -pentaethylenehexamine and higher oligomeric polyethylene amines

The following example illustrates the practice of the invention:

EXAMPLE

Lanthanum acid phosphate (10 $cm^3$ of -12 to -18 mesh particles) is charged to a fixed bed tubular reactor (18 $cm^3$ total volume) and is overlaid with crushed vicor (5 $cm^3$ of -12 to -18 mesh particles). The reactor is heated to 265°C. in an insulated air oven. A mixture of ethylenediamine, monoethanolamine and ammonia (mole ratio EDA:MEA:$NH_3$ is 1:1:6.9), plus 20 weight percent of water (based on the EDA and MEA), is passed over the catalyst at 1400 p.s.i.g. at a liquid hourly space velocity of 3.5 $hr^{-1}$, based on the EDA and MEA. Analysis of the cooled reaction product by gas/liquid chromatography indicates substantial production of predominantly noncyclic polyamines for a long time after a significant decrease in the production thereof has occurred if no water is continuously fed into the reactor.

## Claims

1. Method for enhancing the catalytic activity retention of a phosphorus acid salt catalyst during the production of polyalkylene polyamines in a reaction zone from the reaction of a feed of (i) a reactive nitrogen-containing compound selected from the group consisting of ammonia, a primary amine and a secondary amine, and/or an alkyleneamine compound having at least two amino groups and (ii) an alkanolamine having at least one amino group, comprising maintaining sufficient water in said feed to enhance said catalytic activity retention of said phosphorus acid salt catalyst, said reaction being conducted in the gas phase, in the presence of a catalytically effective amount of said phosphorus acid salt catalyst and at a temperature sufficient to form said polyalkylene polyamines.

2. The method as claimed in Claim 1 wherein enough water is added to said feed to provide said sufficient amount of water in said feed.

3. The method as claimed in Claim 2 wherein, before said feed is fed into the reaction zone, said feed contains an amount of water which is insufficient to provide said sufficient amount of water in said feed and wherein enough additional water is added to said feed with said sufficient amount of water.

4. The method as claimed in Claim 1 wherein said water, said ammonia, said alkyleneamine compound and said alkanolamine compound are mixed and said mixture is fed into the reaction zone.

5. The method as claimed in Claim 1 wherein said ammonia, said alkyleneamine compound and said alkanolamine compound are separately fed into the reaction site and wherein enough water is added to said ammonia or said alkyleneamine compound or said alkanolamine compound to provide said sufficient amount of water in said feed.

6. The method as claimed in Claim 1 wherein said sufficient amount of water in said feed is 1 to 50 weight percent, based on the total weight percent, based on the total weight of said alkyleneamine compound and said alkanolamine compound, of water.

7. The method as claimed in Claim 1 wherein said sufficient amount of water in said feed is 10 to 25 weight percent, based on the total weight of said alkyleneamine compound and said alkanolamine compound, of water.

8. The method as claimed in Claim 1 wherein said alkyleneamine compound is ethylenediamine and said alkanolamine compound is monoethanolamine, and wherein said sufficient amount of said water in said feed is 1 to 50 weight percent, based on the total weight of said ethylenediamine and said ethanolamine, of water.

9. The method as claimed in Claim 1 wherein water is separated from the reaction mixture resulting from said reaction and wherein said separated water is recycled and added to said feed to provide said sufficient amount of water in said feed.

10. The method as claimed in Claim 9 wherein said separated water is in the form of an azeotropic mixture of (a) water and (b) at least one of said alkyleneamine compound.

11. The method as claimed in Claim 10 wherein said alkyleneamine compound is ethylenediamine and said alkanolamine compound is monoethanolamine.

12. The method as claimed in Claim 1 wherein said phosphorus acid salt catalyst is a metal phosphate catalyst.

13. The method as claimed in Claim 1 wherein said metal phosphate catalyst is a metal acid phosphate catalyst.

14. The method as claimed in Claim 13 wherein said metal acid phosphate catalyst is a solid, insoluble, metal acid phosphate catalyst.

15. The method as claimed in Claim 14 wherein said metal acid phosphate catalyst is a Group IIIB metal acid phosphate catalyst, a Group IIIB metal acid monohydrogen phosphate catalyst or a Group IIIB metal acid dihydrogen phosphate catalyst.

16. The method as claimed in Claim 15 wherein the Group IIIB metal is scandium, yttrium, lanthanum or a rare earth lanthanide having an atomic number from 58 to 71.

17. The method as claimed in Claim 13 wherein said metal acid phosphate catalyst is supported on a carrier.

18. The process as claimed in Claim 13 wherein said metal acid phosphate is a Group IIA metal acid phosphate catalyst or a Group IVB metal acid phosphate catalyst.

19. The method as claimed in Claim 1 wherein said reaction is conducted in the vapor phase.

20. The method as claimed in Claim 1 wherein reaction is conducted in the supercritical phase.

21. The method as claimed in Claim 1 wherein said reaction is conducted at a pressure of about 200 to 2,000 p.s.i.g.

22. The method as claimed in Claim 1 wherein said reaction is conducted at a temperature high enough to keep the reactants above their dew points.

23. The method as claimed in Claim 1 wherein a nonaqueous, gaseous diluent is also fed in the reaction zone.

24. The method as claimed in Claim 23 wherein said diluent is selected from the groups consisting of hydrogen, nitrogen, argon, methane, helium, and mixtures of at least two members of such groups.

25. The method as claimed in Claim 1 wherein the liquid hourly space velocity of said reactants is between about 0.1 and about 100 per hour.

26. The method as claimed in Claim 1 wherein said reaction is conducted at a temperature between 260° and 300°C.

27. The process as claimed in Claim 1 wherein said reaction is a continuous reaction.

28. The process as claimed in Claim 1 wherein the feed contains ammonia.

29. The process as claimed in Claim 28 wherein said ammonia is present in an amount in excess of the molar amount participating in said reaction.

30. The process as claimed in Claim 28 wherein he molar ratio of ethylenediamine to ethanolamine to ammonia is 0.25-4/1/5-12.

31. Method for increasing the catalytic activity of a phosphorus acid salt catalyst during the production of polyalkylene polyamines from the reaction of a feed of (i) a reactive nitrogen-containing compound selected from the group consisting of ammonia, a primary amine and a secondary amine, and/or an alkyleneamine compound having at least two amino groups and (ii) an alkanolamine having one amino group, said catalytic activity of said phosphorus acid salt catalyst having decreased during said reaction, comprising incorporating sufficient water in said feed to increase said catalytic activity of said phosphorus acid salt catalyst, said reaction being conducted in the gas phase, in the presence of a catalytically effective amount of phosphorus acid salt catalyst and at a temperature sufficient to form said polyalkylene polyamines.

32. Method as claimed in Claim 31 wherein enough water is added to said feed to provide said sufficient amount of said water in said feed.

33. The method as claimed in Claim 31 wherein, before said feed is fed into the reaction zone, said feed contains an amount of water which is insufficient to provide said sufficient amount of water in said feed and wherein enough additional water is added to said feed with said sufficient amount of water.

34. The method as claimed in Claim 31 wherein said water, said ammonia, said alkyleneamine compound and said alkanolamine compound are mixed and said mixture is fed into the reaction zone.

35. The method as claimed in Claim 31 wherein said ammonia, said alkyleneamine compound and

said alkanolamine compound are separately fed into the reaction zone and wherein enough water is added to said ammonia or said alkyleneamine compound or said alkanolamine compound to provide said sufficient amount of water in said feed.

36. The method as claimed in Claim 31 wherein said sufficient amount of water in said feed is 1 to 50 weight percent, based on the total weight of said alkyleneamine compound and said alkanolamine compound, of water.

37. The method as claimed in Claim 31 wherein said sufficient amount of water in said feed is 10 to 25 weight percent, based on the total weight of said alkyleneamine compound and said alkanolamine compound, of water.

38. The method as claimed in Claim 31 wherein said alkyleneamine compound is ethylenediamine and said alkanolamine compound is monoethanolamine, and wherein said sufficient amount of said water in said feed is 1 to 50 weight percent, based on the total weight of said ethylenediamine and said ethanolamine, of water.

39. The method as claimed in Claim 31 wherein water is separated from the reaction mixture resulting from said reaction and wherein said separated water is recycled and added to said feed to provide said sufficient amount of water in said feed.

40. The method as claimed in Claim 39 wherein said separated water is in the form of an azeotropic mixture of (a) water and (b) at least one of said alkyleneamine compound.

41. Method as claimed in Claim 31 wherein, after said catalytic activity of said phosphorus acid salt catalyst is increased, enough water is continued to be added to said feed so as to provide sufficient water in said feed to maintain said catalytic activity of said phosphorus acid salt catalyst.

42. The method as claimed in Claim 31 wherein said phosphorus acid salt catalyst is a metal phosphate catalyst.

43. The method as claimed in Claim 42 wherein said metal phosphate catalyst is a metal acid phosphate catalyst.

44. The method as claimed in Claim 43 wherein metal acid phosphate catalyst is a solid, insoluble, metal acid phosphate catalyst.

45. The method as claimed in Claim 43 wherein said metal acid phosphate catalyst is a Group IIIB metal acid phosphate catalyst, a Group IIIB metal acid monohydrogen phosphate catalyst or a Group IIIB metal acid dihydrogen phosphate catalyst.

46. The method as claimed in Claim 45 wherein the Group IIIB metal is scandium, yttrium, lanthanum or a rare earth lanthanide having an atomic number from 58 to 71.

47. The method as claimed in Claim 31 wherein said reaction is conducted in the vapor phase.

48. The method as claimed in Claim 31 wherein said reaction is conducted in the supercritical phase.

49. The method as claimed in Claim 32 wherein the liquid hourly space velocity of said reactants is between about 0.1 and about 100 per hour, wherein said reaction is conducted at a temperature between 260° and 300°C., and wherein said reaction is conducted at a pressure of about 200 and 2,000 p.s.i.g.